# EUROPEAN PATENT SPECIFICATION

(11) **EP 1 928 293 B1**
(45) Date of publication and mention of the grant of the patent: **05.09.2018**
(21) Application number: 06794602.0
(22) Date of filing: 29.09.2006
(51) Int. Cl.: A61B 1/00, A61B 1/303, A61B 1/015

(54) **VAGINAL SPECULUM ARRANGEMENT**
VAGINALE SPEKULUMANORDNUNG
SPÉCULUM VAGINAL

(30) Priority: 29.09.2005 EP 05386023
(43) Date of publication of application: 11.06.2008
(73) Proprietor: Dysis Medical Limited, London ECeM 8AL (GB)
(72) Inventor: BALAS, Konstantinos, 153 41 Athens (GR)
(74) Representative: Boult Wade Tennant LLP
(86) International application number: PCT/GB2006/003648
(87) International publication number: WO 2007/036744

(56) References cited:
- WO-A-93/19678
- WO-A-2005/055819
- FR-A- 2 328 440
- US-A- 3 789 829
- US-A- 5 458 595
- US-A1- 2002 197 728
- US-A1- 2003 207 250
- US-A1- 2003 225 313
- US-A1- 2005 090 751
- US-B1- 6 432 049

## Description

### FIELD OF THE INVENTION

The present invention relates to medical devices, in particular to speculums. More particularly, the present invention relates to a vaginal speculum couplable to or which incorporates an applicator for the uniform delivery of a standardized dose of a liquid diagnostic marker onto a woman's lower genital tract.

### BACKGROUND OF THE INVENTION

Detection and identification of pathologic alterations of the woman's low genital tract (cervix of the uterus, vagina) involves a series of medical procedures including screening tests (pap-test), tissue examination with the aid of a microscope (colposcopy), biopsy sampling and histology. An abnormal pap-test is followed by colposcopy, where the vagina is opened with the aid of a speculum to allow tissue visualization with the aid of a microscope. In colposcopy, a number of diagnostic markers are applied topically, which alter the optical properties of the tissue, depending on the pathology. Particularly, application of 3-5% acetic acid solution provokes a reversible whitening of the abnormal tissue areas. It has been proved that the degree and the duration of the whitening effect correlates well with the neoplasia grade. The provoked contrast enhancement between normal and abnormal areas provide a valuable means for assisting colposcopic diagnosis and for locating abnormal areas for biopsy sampling and treatment. There is a considerable confidence for the diagnostic value of these diagnostic markers, which has been developed during the 70 years usage of these tests in the clinical practice.

The employment of marker-based *in vivo* tests as an alternative to the *in vitro* pap test for screening cervical pathology has increased in recent years. Marker-based *in vivo* tests employ a procedure similar to the colposcopy procedure, but typically are performed without the use of a microscope (colposcope). The vagina is opened with the aid of a speculum, which is followed by the application of acetic acid solution onto tissue surface and naked-eye monitoring of the marker-induced alterations in the colour of the examined tissue. This technique is known as speculoscopy. In contrast to the pap-test, speculoscopy offers diagnostic results immediately, which enable the biopsy sampling and/or the treatment of the lesion even during the same consultation.

One main drawback of both colposcopy and speculoscopy arises from the fact that the quantity of applied marker is not standardized, while the marker administration means and procedures do not ensure its uniform application over the entire area of the examined tissue. In addition, the injection means employed obstruct the rear opening of the speculum, not allowing the monitoring of the effects provoked by the marker during its application and due to this fact critical diagnostic information is missed. Typically, an uncontrolled volume of the marker is applied either by washing the tissue with the aid of a cotton brush, moistened with acetic acid solution, or with the aid of a general purpose, hand held atomizer, which delivers a random quantity of the marker remotely. In some cases more than one injection are performed in a repetitive manner during the evolution of the acetowhitening phenomenon in order to achieve better contrast.

Clinical research, conducted by the inventor of the present invention, has shown that the monitoring of the effects provoked by the marker, during and after its application, has a great diagnostic value. The same research has also shown that the concentration and the quantity of the marker solution, applied onto the examined tissue are very critical since for a given pathology, different marker doses generate different optical effects, which may cause misdiagnosis. Particularly, for a given tissue pathology, an insufficient marker dose may cause in cancerous lesions an acetowhitening pattern similar to the one provoked by an optimum marker quantity in inflammations and in low grade neoplasias. Similarly, a high marker dose can cause an acetowhitening pattern in inflammations and low grade precancerous lesions typically found in cancerous lesions. Consequently, the lack of an arrangement enabling the standardization of the marker quantity applied onto the tissue surface may result in false positive and/or false negative results, thus, diminishing the diagnostic performance of these tests in terms of both sensitivity and specificity.

A number of prior art documents disclose various speculum arrangements with imaging and illuminations means integrated with a speculum, but they are characterized by the lack of injection means for applying uniformly a standardized quantity of a diagnostic marker, while simultaneously allowing for the inspection of the optical effects produced by the latter.

Such prior art documents include GB214913 and GB191027965. These documents disclose a vaginal speculum with incorporated fluid injection means. The purpose of fluid injection means, as described in these documents, is for washing the woman's low genital tract and it does not offer any standardization of the injected liquid. It is worth noticing that in these prior art documents, washing does not employ a diagnostic marker and therefore it is not intended to assist diagnosis and screening. More importantly, it does not allow for the visualization of the area of interest, since the whole inner space of the speculum is occupied by the fluid injection means and no free space is available allowing observation and insertion of treatment tools.

Other prior art documents disclose vaginal specula with integrated illumination means for illuminating the vagina. Such specula are disclosed, e.g., in documents GB1408382, US3762400, US3851642. These vaginal specula are intended for the medical examination of the vagina, but they are not accompanied with integrated fluid injection means, necessary for a diagnostic medical examination of the vagina wherein the uniform application of a standard volume of a diagnostic marker is necessary.

Other prior art described in documents WO9007299, WO9728753, US4210133 and US4046140, discloses vaginal specula with an integrated microscope or camera for observing and/or for capturing images of the cervical tissue. In the described implementations the microscopes or cameras are located within the blades not allowing the insertion of tools for biopsy sampling and treatment simultaneously with the inspection with the aid of a microscope or camera. In addition, the instruments disclosed in the foregoing documents do not allow the injection of diagnostic markers. The prior art document US20040122327 discloses an uteroscope arrangement including a panoramic lens for viewing the entire uterine cavity in one image that is mounted on an elongated shaft for insertion into the patient's uterus. One or more transparent inflatable balloons are mounted on the elongated shaft surrounding the optical imaging system. An instrument channel is provided in the shaft of the uteroscope for insertion of instruments, such as a suction tube, external to or in between the transparent inflatable balloons.

US 2005/0090751 relates to a method and an apparatus for the in vivo, non-invasive, early detection of alterations and mapping of the grade of these alterations, caused in the biochemical and/or in the functional characteristics of epithelial tissues during the development of tissue atypias, dysplasias, neoplasias and cancers.

### OBJECTS OF THE INVENTION

One object of the present invention is to provide a speculum arrangement, integrating means for dispensing uniformly a standardised marker volume, while simultaneously allowing for the visualization and monitoring of the provoked optical effects, for diagnostic and screening purposes and the insertion of treatment tools into the vaginal canal, for biopsy sampling and treatment.

It is another object of the present invention to provide a speculum arrangement further integrating optical, electronic imaging, and illumination means with a speculum arrangement embodying a diagnostic marker injection mechanism, while simultaneously allowing for the insertion of treatment tools into the vaginal canal.

It is still another object of the invention to provide a speculum arrangement with an extension shaft, which may be connected with a mechanical support and positioning means, including the ones employed in imaging devices used in colposcopy, for the support and stabilization of the speculum and hands free operation.

### SUMMARY OF THE INVENTION

The present invention is defined in the claims and relates to a vaginal speculum embodying an applicator for the uniform delivery of a standardized dose of a liquid diagnostic marker onto the woman's lower genital tract. The applicator comprises of a marker container and a mechanism for transferring a desirable quantity of its content to an injection probe for dispensing the marker onto the tissue surface. The probe may be a nozzle generating a desirable injection pattern, depending on the location of the tissues to be examined. The cross section of the injection probe is substantially smaller than the rear opening of the speculum, so that the monitoring of the optical effects provoked to the tissue by the marker and the insertion of treatment tools is not obstructed.

The probe may be affixed to an extension rod, which may be mechanically coupled with the speculum blades, in such a way that the longitudinal axis of the probe and consequently the injection direction remains stable, independently from the actual opening angle of the blades, determined by the anatomy of the vaginal wall. Optical, electronic imaging means, illumination means and treatment tools may be mounted onto the extension rod, which rod may be detachably attached to mechanical positioning systems or to imaging devices used in colposcopy.

The disclosed speculum arrangement may be used as a tool for diagnostic and screening examinations and for the treatment of cervical and vaginal neoplasias.

Thus, according to a first aspect of the invention there is provided a vaginal speculum arrangement comprising, a blade system for opening the vagina having a first blade (1) and a second blade (2) positionable relative to each other in a plurality of angles and a longitudinal symmetry axis (10) between a distal portion and a proximate portion of each of the first (1) and second blades (2), and a mechanical support having a shaft (8) with a first shaft end mechanically coupled with the blade system and second shaft end detachably couplable to an injection mechanism, a support member or an imaging apparatus, characterised in that said first shaft end of said shaft (8) is jointed with a blade-handle joint of the first blade (6), and a structure of the blade-handle joint of the second blade (6) moves along a structure formed along a longitudinal axis of said shaft (8).

The second shaft end may be detachably coupled to an injection mechanism for dispensing a diagnostic marker onto the surface of the examined tissue comprising an injection probe (7) having a longitudinal axis (11), a marker container (15,16) and a means for enabling injection of the marker (18), wherein the dimensions of the cross section of the injection probe (40) are substantially smaller than the dimensions of the cross section (41) of a rear aperture (42) of the blade system , wherein the relative position of the longitudinal axis of the injection probe (11) and the longitudinal symmetry axis of the blade system (10) remain substantially fixed for each of the plurality of angles between the first and second blades and wherein application of the diagnostic marker by the injection probe is not influenced by separation of the first and second blades and thus the injection probe allows for a substantially homogeneous application of the diagnostic marker on a desired area in the examined vaginal or cervical tissue, irrespective of the degree of separation of the blades. Thus, the construction allows for easy observation of the desired area through the rear aperture of the blade system, before during and after the injection of the diagnostic marker. This is readily achieved by ensuring that the dimensions of the cross section of the injection probe are substantially smaller than the dimensions of the cross section of the rear aperture of the blade system.

Any suitable means for enabling injection of the marker may be employed.

As is described in more detail above, the lack of an arrangement enabling the standardization of the marker quantity applied onto the tissue surface may result in false positive and/or false negative results, thus, diminishing the performance of diagnostic tests in terms of both sensitivity and specificity. The present invention addresses this problem by ensuring that application of the marker through the injection system is not influenced by the movement of the speculum when opening the vagina. Thus, no matter what in use position the speculum adopts (depending upon the anatomy of the individual under investigation), the injection system is still able to deliver the diagnostic marker to a standard area of tissue.

The relative position of the longitudinal axis of the injection probe and the longitudinal symmetry axis of the blade system remain substantially fixed for each of the plurality of angles between the first and second blades due to the nature of the mounting of the injection probe on the speculum. Movement of the blades is controlled by movement of corresponding handles which are connected to the blades and which are manipulated by the user of the device. In one embodiment, the second shaft end is detachably coupled to the injection mechanism. In a further embodiment, the second shaft end is detachably coupled to a support member or to an imaging apparatus. The support member may include an articulated arm with a first end portion affixed to a base and a second end portion affixed to a locking mechanism of the shaft. In a still further embodiment, the injection probe is mounted on a portion of the shaft.

As defined in the claims, the first shaft end of said shaft is jointed with a blade-handle joint of the first blade. In one embodiment, a pin of the blade-handle joint of the second blade moves within a groove, formed along a longitudinal axis of said shaft. According to alternative constructions, the groove may be replaced by any other suitable structure such as a slot for example. Thus, the term "groove" is intended to encompass all functional equivalents. Likewise the pin may be replaced by any other type of structure which is moveable (in stable fashion) along the longitudinal axis of the shaft and thus the term "pin" is intended to encompass all functional equivalents. By way of example, as the blades of the speculum open symmetrically around the speculum's pivoting joint, the probe is mounted on, either directly or indirectly, the pivot point. Additional structures may be fixed onto the shaft in order to position the injection probe appropriately as would be readily appreciated by the skilled person.

Whilst the application of diagnostic marker from the injection probe is not influenced by the relative movement of the blades according to the invention, the injection probe may nevertheless be capable of independent movement. Thus, for example, if the blades move vertically and thus remain parallel to one another, the injection probe may be mounted such that it remains in a fixed location between the two blades independent of their degree of separation. This may be achieved through use of a suitable gearing mechanism or a rack and pinion mechanism for example. In a further embodiment, the injection probe may be rotatably mounted on the speculum such that its orientation can be modified manually but remains fixable and independent of the movement of the speculum blades.

In one preferred embodiment according to the invention, the shaft of the vaginal speculum arrangement comprises a locking mechanism. This locking mechanism may be a separate member that interacts with the shaft to connect the speculum to additional components. In one embodiment, the locking mechanism includes one of a mechanical locking mechanism or a magnetic or an electromagnetic locking mechanism.

In a further embodiment the injection probe is affixed to said mechanical support in the vicinity of said locking mechanism.

In a preferred construction, the injection probe comprises a nozzle. Preferably the nozzle comprises a needle nozzle.

The injection mechanism may comprise a hydraulic pump means for pumping a predetermined volume of a marker into and through the injection probe. Preferably, the predetermined volume of the marker ranges between about 2.5 ml and about 3.5 ml. The marker may be any suitable marker for use in visualization of the tissue of interest. In a preferred embodiment, the marker is acetic acid. Preferably, the marker is between about 3% and about 5% acetic acid solution.

In a further embodiment, the vaginal speculum arrangement of the invention further comprises a light source. The light source may be affixed to the support member in a vicinity of the shaft locking mechanism.

The vaginal speculum arrangement of the invention may further comprise an optical element. The optical element may be any of a magnifying optical element, a focusable optical element, an optical filter or a pair of polarizers, one for polarizing the light emitted by the light source and one for polarizing the light reflected by the tissue, having their polarization axes perpendicular to each other.

Any of the components of the vaginal speculum arrangement, in particular the blade system/shaft/injection probe may be formed from a metallic material. The component parts may be re-usable. In an alternative embodiment, some or all of the components of the vaginal speculum arrangement, in particular the blade system/shaft/injection means include a portion formed from a polymeric compound, such as a plastics material for example. Such components are preferably disposable.

### BRIEF DESCRIPTION OF THE DRAWINGS

Figure 1 illustrates a vaginal speculum arrangement composed by blades and handles, an extension rod mechanically coupled with the blades, an injection probe affixed onto the rod, a liquid marker container, and hydraulic means to enable injection of the marker.
Figure 2 illustrates a vaginal speculum arrangement composed of blades and handles, an extension rod mechanically coupled with the blades, and a mechanical support attached to a platform such as the ground or to the examination bed and connected detachably with the extension shaft. Onto the mechanical support and in the vicinity of its connection point with the extension shaft, the following components are mounted: an injection probe, a light source with a removable polarizer and removable rotating imaging polarizer, optical filter means and image magnifying optics.
Figure 3 illustrates a rear-view of a speculum in accordance with the teachings of the present invention.
Figure 4 illustrates a needle nozzle with a needle having an outside diameter sized to maximize the field-of-view through the rear aperture of a speculum in accordance with the teachings of the present invention. A coupling mechanism is used for connection of the needle nozzle with the tube providing a channel through which the marker flows.
Figure 5 illustrates an apparatus for securing a speculum shaft onto an optical imaging system, or onto a base member.
Figure 6 is a perspective view of the locking mechanism of Figure 5 showing the cam action wedge and its housing in the locking mechanism.
Figure 7 illustrates an alternative apparatus for securely connecting a speculum shaft to a support member, such as a base, or to an optical imaging system for example.

### DETAILED DESCRIPTION OF THE INVENTION

A Cusco-type speculum is illustrated in the figures for illustrative purposes. Those skilled in the art will appreciate the present disclosure is not limited to such a speculum, but rather is applicable to any kind of speculum having a mechanical arrangement suitable for opening the vagina to enable the visualization of the tissues composing a woman's lower genital tract.

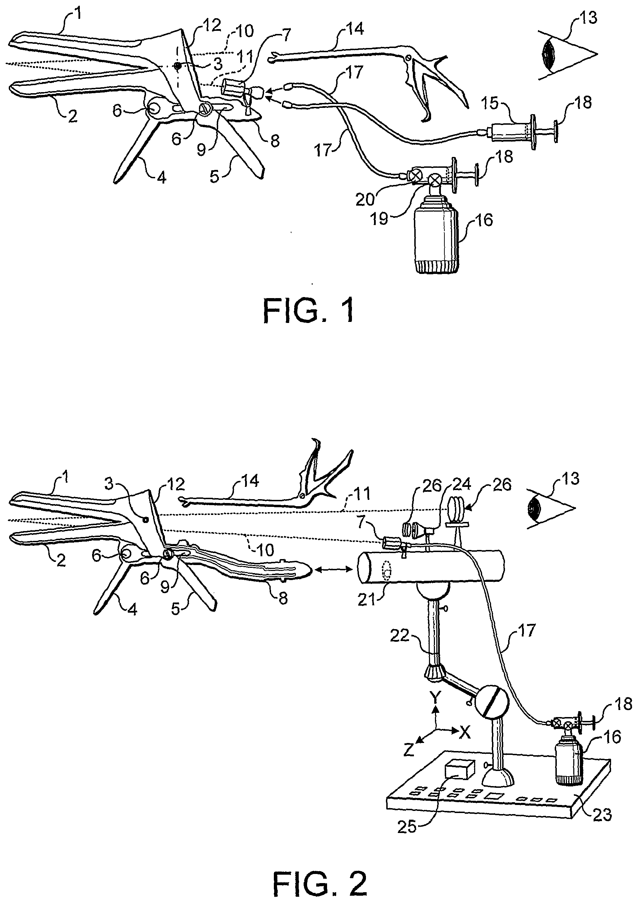

Figure 1 depicts a Cusco-type speculum having two blades (1, 2) connected to each other with the aid of a pivoting joint (3), located at the rear part of the blades. Each blade is jointed with the corresponding handle (4, 5) with the aid of a pin (6). The separation distance between the handles (4, 5) becomes maximum when the front parts of the blades (1, 2) are in contact. When the speculum is inserted into the vagina, the blades are in or near contact to each other, for the patient's comfort. After insertion, the handles (4, 5) are approached to each other, separating the blades (1, 2) and opening the vagina. The blade separation is mechanically locked at a desirable position, determined by the anatomy of the tissue. Then the examination follows involving the application of one or more diagnostic markers and the monitoring of the marker-induced alterations in the properties, e.g., the colour, of the tissue. As has been stated above, the uniform application of a standardized quantity of the diagnostic marker, while simultaneously allowing for the tissue inspection is critical for examination and diagnostic evaluation.

Uniform and simultaneous application of the marker over the entire area of the examined tissues can be achieved with the aid of a liquid injection mechanism, capable of dispensing the marker from a distance. In the case that the cervix of the uterus is examined, and because of its almost circular shape, a preferable injection pattern is conical with a maximum diameter equal with the diameter of the cervix, which is approximately 2.5-3 cm (1 inch).

An injection probe (7) is preferably mounted properly onto a fixed position, so that its injection direction is not affected by the opening angle of the speculum blades (1, 2), which may vary due to the anatomy of the vagina. Such a fixed position cannot be achieved by affixing the injection probe on any of the blades, since by changing their angle the injection direction will change accordingly. Consequently, depending on the blade angle different parts of the tissue will be exposed to a different volume of the marker fluid.

In the case of a Cusco-type speculum the blades open symmetrically around the speculum's pivoting joint (3) which is thus an eligible mount upon which to affix the injection probe (7). However, according to an example, the injection probe (7) is affixed onto an extension shaft (8), which is mechanically coupled with the pins (6) connecting the handles with the blades. The front part of the shaft is jointed with the blade-handle joint of the first blade (4), while the pin of the blade-handle joint of the second blade (5) can slide within a groove (9), formed along the longitudinal axis of the extension shaft (8). This arrangement ensures that the relative position of the longitudinal axis of the probe (10) with respect to the longitudinal symmetry axis of the blade system (11) remains the same for all possible blade angles. Therefore, by properly mounting the injection probe (7) onto the extension shaft (8), its longitudinal axis intersects the central area of the examined tissue in all possible relative positions of the blades (1, 2), thus ensuring uniform application of the marker in various anatomic conditions.

In one example, the injection probe (7) is a nozzle remotely delivering a mist of liquid marker droplets of a desirable size onto the surface of the tissue. The cross section of the injection probe (7) is substantially smaller that the rear opening of the blade system (12) and preferably it has a needle nozzle-like shape for the purpose of not obscuring the visualization (13) of the tissue before, during and after injection and for allowing for the insertion of treatment tools (14). The liquid marker is transmitted to the injection probe (7) from a marker container (15, 16) either by permanently or detachably connecting these parts to each other, or through a tube (17) connecting these parts either permanently or detachably. The injection of the fluid is achieved with the aid of hydraulic pressure manually or otherwise applied.

In one example, the container and the hydraulic means comprise a syringe with a container (15) and a piston (18). In another example, the container is a bottle (16) and the hydraulic means is a tube with two one-way valves (19 and 20) and a piston (18). When the piston (18) is pulled out, the liquid fills-up the tube enclosing the piston with a desirable quantity of marker liquid and the valve of the bottle (19) closes. By pushing the piston in, the tube valve (20) opens, the bottle valve (19) closes and the liquid is injected from the injection probe (7). In one example, more than one marker staining different features of diagnostic relevance is performed with the arrangement described above either simultaneously or in time sequence.

Clinical investigations conducted by the inventors of the present invention have shown that the optimum quantity of the marker is a volume of between about 2.5 ml and 3.5 ml. This volume ensures a sufficient and uniform washing of the entire surface of the cervix to produce the diagnostic optical effect. At the same time, this volume is desirable, since it eliminates unwanted accumulation of marker in excess between the lower blade (2) and the lower part of the examined tissue, which may obscure the visualization of the tissue.

The vaginal speculum arrangement of the present invention, as illustrated in Figure 1, may be manufactured either in part or in whole either from metallic or from synthetic (plastic, Plexiglas) material. An exemplary speculum arrangement either in part or in whole, may be either re-usable or disposable. In one embodiment, the speculum arrangement comprising the blade and handle system, the extension shaft onto which the nozzle is affixed, the nozzle mechanically coupled with the syringe pre-filled with the marker, is disposable.

Figure 2 depicts another example of the vaginal speculum arrangement. The length of the extension shaft (8) is determined by the working distance of the optical imaging apparatus employed for the examination the lower part of woman's genital system, such as cameras, colposcopes etc. and combinations thereof. The extension shaft is detachably connected with these imaging apparatuses, with the aid of a locking mechanism (21). The locking mechanism (21) is affixed onto the imaging apparatus and at a proper location so that when the locking mechanism is coupled with the extension shaft, the longitudinal symmetry axis of the blade system (11) coincides substantially with the bisector of the viewing angle (13). In another example of this invention, suitable for speculoscopy use, the locking mechanism (21) is mounted on a mechanical support, which in turn is either affixed onto the examination bed or includes a base (23) placed on the ground. The mechanical support may be an articulating arm (22) to facilitate manipulations for the connection of the speculum shaft (8) with the locking mechanism (21).

Onto the mechanical support which may be an articulating arm (22), and in the vicinity of its connection point with the extension shaft (8), the following components may be mounted: an injection probe (7), connected with a marker container (15, 16) either directly or through a tube (17) and hydraulic means for enabling injection, all having the specifications described above with respect to Figure 1, a light source (24) with a power supply (25) and at least one of the following optical elements (26) interposed in the illumination and imaging ray paths: magnifying and focusing optics, filters and polarizers. The optical elements (26) may be mounted in a removable manner from the path of the rays, by tilting them left or right. The polarizers may be affixed on a mount allowing the rotation of their polarization axes.

The cross section of the light source (24) and illumination optics (26) is substantially smaller than the rear optical aperture of the blade system (12) for the purpose of not obscuring the visualization of the tissue.

The light source (24) may be a halogen lamp and/or a LED lamp or other suitable light source. When the polarization axis of the imaging polarizer becomes, after rotation, vertical with the polarization axis of the light source, then the surface reflection (glare) is eliminated, resulting in a substantial improvement of the perceived contrast. This facilitates the detection and monitoring of features of diagnostic importance. The perceived contrast is further enhanced with the aid of an optical filter and image magnifying means (26).

Once the extension shaft (8) is connected with the mechanical support, the longitudinal axis of the injection probe may have a fixed relative position with the longitudinal axis (11) of the blade system, ensuring that the former intersects the central area of the tissue and the uniform application of the marker onto the entire area of the examined tissue.

The vaginal speculum arrangement of the current invention, illustrated in figure 2, may be manufactured either in part or in total either from metallic or from synthetic (plastic, Plexiglas) material. The speculum arrangement of the current invention may be in part or include either re-usable or disposable. In one preferable example of the vaginal arrangement as depicted in Figure 2, the blade-handle system with the extension shaft is disposable and the mechanical mount with the components (in part or in whole) mounted on it, is re-usable.

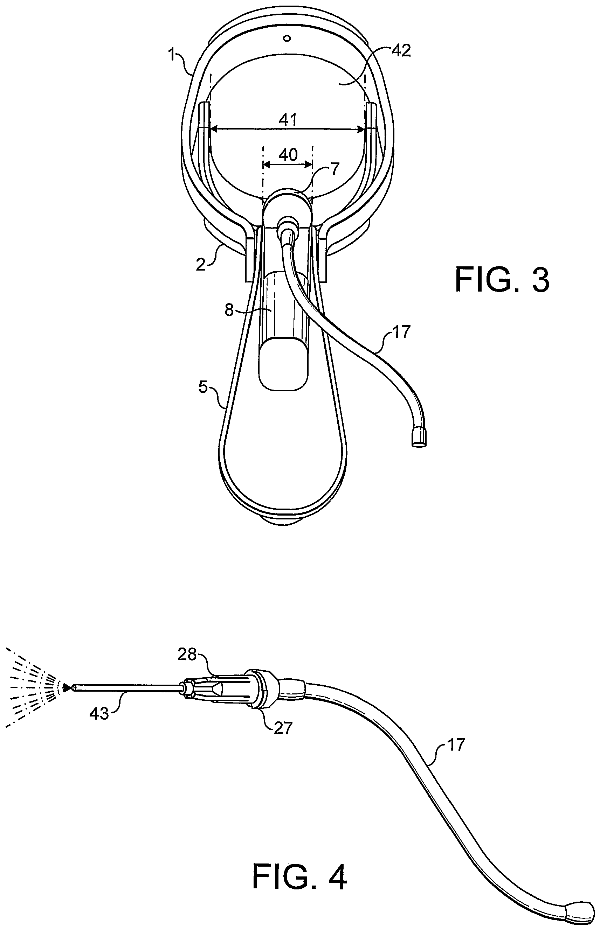

Figure 3 illustrates a rear-view of the joined speculum blades (1, 2), the extension shaft (8) and the nozzle (7). The dimensions of the cross section (40) of the nozzle are substantially smaller than the dimensions of the cross section (41) of a rear aperture (42) of the blade system, thus allowing for the visualization of the examined area before, during, and after the injection of the marker.

Figure 4 illustrates a needle nozzle (27), with the needle (43) having an outside diameter sized to maximize the field-of-view through the rear aperture (42) of the blade system. A coupling mechanism (28) is used for the connection of the needle nozzle (27) with the tube (17) providing a channel for the marker from a container holding the marker to an input orifice of the coupling means (28).

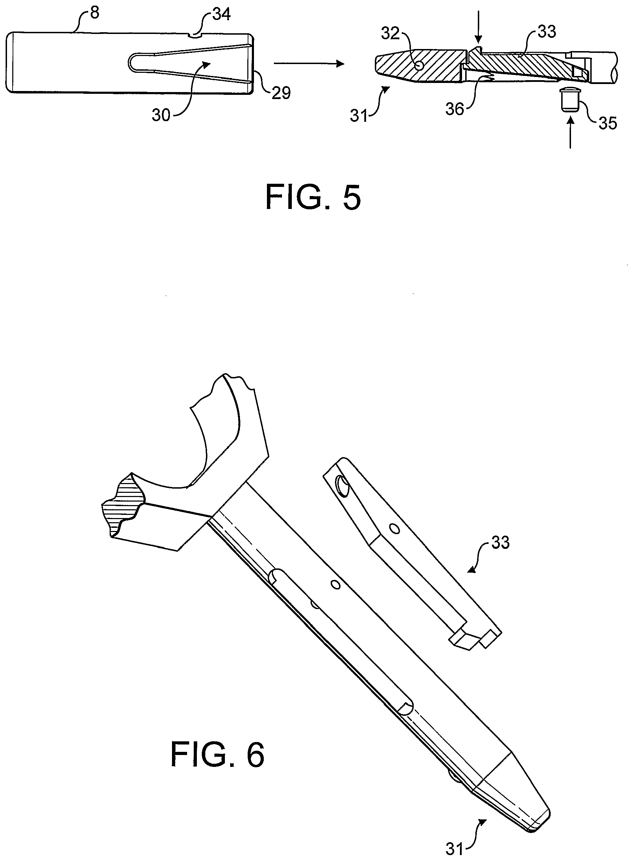

Figure 5 and 6 illustrate one example of the shaft (8) in more detail. Those skilled in the art will appreciate the shaft (8) is not limited to the example illustrated and other shaft configurations are possible. The shaft (8) illustrated in Figure 5 is well suited for use in securing the speculum shaft onto an optical imaging system (26), onto a base member (23), or both. In the illustrated example, the distal end (29) of a speculum shaft (8) includes a conically tapered slot (30) in a bottom side. The conically tapered slot (30) acts as a guide for the proper alignment of the speculum with respect to the external optical system (26). A securing mechanism engages with the distal end (29) of the shaft (8) with an extension pin (31) that has a dowel pin (32) having a longitudinal axis perpendicular to the longitudinal axis of the extension pin (31). The position of the dowel pin (32) determines the displacement of the speculum from the external optical system. The distal end (29) of the shaft (8) engages with the extension pin (31) using a spring-loaded, cam action wedge (33). The distal end (29) of the shaft (8) also includes a receptacle slot (34) to mate with the cam action wedge (33).

In operation, the shaft (8) is moved towards the optical system to urge the dowel pin (32) into contact with the conically tapered groove (30) in the shaft (8) until the cam action wedge (33) mates with the receptacle slot (34) in the shaft (8).

The shaft (8) is unlocked from the dowel pin (32) by pressing on a release button (35) which has the effect of engaging with the cam action wedge (33). In this state, the receptacle slot (34) is devoid of a locking member and the shaft (8) can be removed. The cam action wedge (33) and the release button (35) are returned to their normal states due to the action of a spring (36) housed in the engagement pin (31).

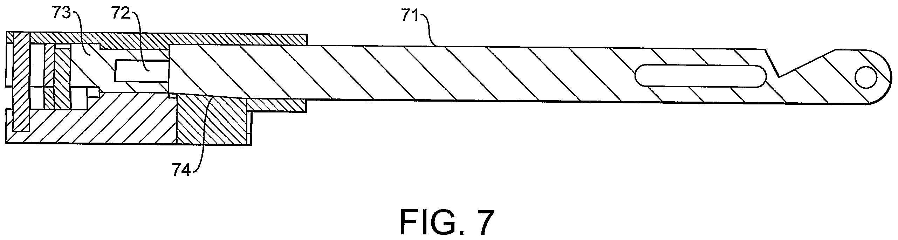

In one embodiment, and as shown in Figure 7, the vaginal speculum may be attached to the support with the use of a magnetic locking mechanism. The mechanism may consist of a connecting shaft (71) with a ferrite insert at the distal end (72) and a corresponding magnet (73) housed in the support. The said magnet (73) may be a permanent magnet, or in another embodiment, an electromagnet.

The support is designed with a conical inlet (74) in order to provide accurate alignment, ease of engagement and elimination of unwanted clearances at the junction of the connecting shaft and the support. In the shown arrangement, the magnet (73) is a permanent magnet. In this arrangement, the vaginal speculum connected to the connecting shaft (71) is brought into the proximity of the permanent magnet by using the guidance provided by the conical opening in the support. Following usage, the connecting shaft is de-coupled from the support by using an ambidextrous release trigger (not shown) and pulling it. The pivot action of the trigger causes the support - connection shaft connection to be severed. The vaginal speculum and the connection shaft may then be extricated from the support.

In another embodiment, the magnet used may be an electromagnet whereby the "circuit" is completed upon contact of the inserted ferrite with the corresponding contact point in the support. The release of the connection in this embodiment may be effected by using a wired button to interrupt the circuit allowing the release of the connection shaft from the support.

## Claims

1. A vaginal speculum arrangement comprising:
a blade system for opening the vagina having a first blade (1) and a second blade (2) positionable relative to each other in a plurality of angles and a longitudinal symmetry axis (10) between a distal portion and a proximate portion of each of the first (1) and second blades (2); and
a mechanical support having a shaft (8) with a first shaft end mechanically coupled with the blade system and second shaft end detachably couplable to an injection mechanism, a support member or an imaging apparatus;
**characterised in that**
said first shaft end of said shaft (8) is jointed with a blade-handle joint of the first blade (6), and a structure of the blade-handle joint of the second blade (6) configured to move along a structure formed along a longitudinal axis of said shaft (8).

2. The vaginal speculum arrangement of claim 1 further comprising an injection mechanism, wherein:
the second shaft end is detachably coupled to an injection mechanism for dispensing a diagnostic marker onto the surface of the examined tissue having an injection probe (7) having a longitudinal axis (11), a marker container (15,16) and a means for enabling injection of the marker (18), wherein the dimensions of the cross section of the injection probe (40) are substantially smaller than the dimensions of the cross section (41) of a rear aperture (42) of the blade system, and
wherein the relative position of the longitudinal axis of the injection probe (11) and the longitudinal symmetry axis of the blade system (10) remain substantially fixed for each of the plurality of angles between the first and second blades, wherein the injection probe (7) allows for a substantially homogeneous application of the diagnostic marker on a desired area in the examined vaginal or cervical tissue, irrespective of the opening angle of the blades (1,2) and allows for observation of the desired area through the rear aperture of the blade system, before during and after the injection of the diagnostic marker.

3. The vaginal speculum arrangement of claim 2, wherein the second shaft end is detachably coupled to a support member or to an imaging apparatus.

4. The vaginal speculum arrangement of claim 2 wherein the injection probe (7) is mounted on a portion of the shaft (8).

5. The vaginal speculum arrangement of any one of claims 3 or 4 wherein the shaft (8) comprises a locking mechanism.

6. The vaginal speculum arrangement of claim 3, wherein the support member includes an articulated arm (22) with a first end portion affixed to a base (23) and a second end portion affixed to a locking mechanism of the shaft (21).

7. The vaginal speculum arrangement of claim 3 or 4, wherein said locking mechanism (21) includes one of a mechanical locking mechanism or a magnetic or an electromagnetic locking mechanism.

8. The vaginal speculum arrangement of claim 5, wherein said injection probe (7) is affixed to said mechanical base in a vicinity of said locking mechanism.

9. The vaginal speculum arrangement of any one of claims 2 to 8, wherein said injection probe (7) comprises a nozzle.

10. The vaginal speculum arrangement of claim 9 wherein said nozzle (7) comprises a needle nozzle (43).

11. The vaginal speculum arrangement of any one of claims 2 to 10, wherein the injection mechanism further comprises a hydraulic pump means (15, 16, 18, 19, 20) for pumping a predetermined volume of a marker into and through said injection probe (7).

12. The vaginal speculum arrangement of claim 11, wherein said predetermined volume of the marker ranges between about 2.5 ml and about 3.5 ml.

13. The vaginal speculum arrangement of claim 11 or 12, wherein said marker is between about 3% and about 5% acetic acid solution.

14. The vaginal speculum arrangement of any one of claims 2 to 13 further comprising a light source (24).

15. The vaginal speculum arrangement of any one of claims 5 to 8, further comprising a light source (24) affixed to the support member in a vicinity of said shaft locking mechanism (21).

16. The vaginal speculum arrangement of any one of claims 2 to 15, further comprising an optical element (26).

17. The vaginal speculum arrangement of claim 16, wherein the optical element (26) comprises one of a magnifying optical element, a focusable optical element, an optical filter or a pair of polarizers, one for polarizing the light emitted by the light source and one for polarizing the light reflected by the tissue, having their polarization axes perpendicular to each other.

18. The vaginal speculum arrangement of any one of claims 2 to 17 wherein said blade system/said shaft (8)/said injection probe (7) are formed from a metallic material.

19. The vaginal speculum arrangement of claim 18 wherein said blade system/said shaft (8) /said injection probe (7) are re-usable.

20. The vaginal speculum arrangement of any one of claims 2 to 17, wherein said blade system/said shaft (8)/said injection means (7) comprise a portion formed from a polymeric compound.

21. The vaginal speculum arrangement of claim 20, wherein said blade system/said shaft (8)/ said injection means (7) are disposable.

## Patentansprüche

1. Vaginalspekulumanordnung, welche umfasst
ein Blattsystem zum Öffnen der Vagina mit einem erstem Blatt (1) und einem zweiten Blatt (2), die relativ zueinander in einer Vielzahl von Winkeln positionierbar sind, und einer Längssymmetrieachse (10) zwischen einem distalen Abschnitt und einem proximalen Abschnitt des ersten (1) und des zweiten Blatts (2); und
eine mechanische Stütze, die einen Schaft (8) mit einem ersten Schaftende, das mechanisch mit dem Blattsystem gekoppelt ist, und einem zweiten Schaftende, das lösbar mit einem Einspritzmechanismus, einem Stützelement oder einer Abbildungsvorrichtung kuppelbar ist, umfasst;
**dadurch gekennzeichnet, dass**
das erste Wellenende des Schafts (8) mit einer Blatt-Griff-Verbindung des ersten Blatts (6) verbunden ist, und eine Struktur der Blatt-Griff-Verbindung des zweiten Blatts (6) konfiguriert ist, um sich entlang einer Struktur zu bewegen, die entlang einer Längsachse des Schafts (8) ausgebildet ist.

2. Vaginalspekulumanordnung nach Anspruch 1, ferner mit einem Injektionsmechanismus, wobei:
das zweite Schaftende mit einem Injektionsmechanismus zur Abgabe eines Diagnosemarkers auf die untersuchte Gewebeoberfläche lösbar gekoppelt ist, der eine Injektionssonde (7) mit einer Längsachse (11), einen Markerbehälter (15, 16) und ein Mittel zum Ermöglichen der Injektion des Markers (18) aufweist, wobei die Abmessungen des Querschnitts der Injektionssonde (40) wesentlich kleiner sind als die Abmessungen des Querschnitts (41) einer hinteren Öffnung (42) des Blattsystems, und
wobei die relative Position der Längsachse der Injektionssonde (11) und der Längssymmetrieachse des Blattsystems (10) für jeden der Vielzahl von Winkeln zwischen dem ersten und dem zweiten Blatt im Wesentlichen fixiert bleibt, wobei die Injektionssonde (7) eine im Wesentlichen homogene Applikation des Diagnosemarkers auf einen gewünschten Bereich im untersuchten vaginalen oder zervikalen Gewebe unabhängig vom Öffnungswinkel der Blätter (1, 2) ermöglicht und die Beobachtung des gewünschten Bereichs durch die hintere Öffnung des Blattsystems vor und nach der Injektion des Diagnosemarkers ermöglicht.

3. Vaginalspekulumanordnung nach Anspruch 2, wobei das zweite Wellenende lösbar mit einem Trägerelement oder einem Abbildungsgerät gekoppelt ist.

4. Vaginalspekulumanordnung nach Anspruch 2, wobei die Injektionssonde (7) an einem Teil des Schafts (8) angebracht ist.

5. Vaginalspekulumanordnung nach einem der Ansprüche 3 oder 4, wobei der Schaft (8) einen Verriegelungsmechanismus aufweist.

6. Vaginalspekulumanordnung nach Anspruch 3, wobei das Stützelement einen Gelenkarm (22) mit einem ersten Endabschnitt, der an einer Basis (23) befestigt ist, und einem zweiten Endabschnitt, der an einem Verriegelungsmechanismus des Schaftes (21) befestigt ist, aufweist.

7. Vaginalspekulumanordnung nach Anspruch 3 oder 4, wobei der Verriegelungsmechanismus (21) einen mechanischen Verriegelungsmechanismus oder einen magnetischen oder einen elektromagnetischen Verriegelungsmechanismus umfasst.

8. Vaginalspekulumanordnung nach Anspruch 5, wobei die Injektionssonde (7) an der mechanischen Basis in der Nähe des Verriegelungsmechanismus befestigt ist.

9. Vaginalspekulumanordnung nach einem der Ansprüche 2 bis 8, wobei die Injektionssonde (7) eine Düse umfasst.

10. Vaginalspekulumanordnung nach Anspruch 9, wobei die Düse (7) eine Nadeldüse (43) umfasst.

11. umfasst Vaginalspekulumanordnung nach einem der Ansprüche 2 bis 10, wobei der Injektionsmechanismus ferner eine hydraulische Pumpeinrichtung (15, 16, 18, 19, 20) zum Pumpen eines vorbestimmten Volumens eines Markers in und durch die Injektionssonde (7) umfasst.

12. Vaginalspekulumanordnung nach Anspruch 11, wobei das vorbestimmte Volumen des Markers zwischen etwa 2,5 ml und etwa 3,5 ml liegt.

13. Vaginalspekulumanordnung nach Anspruch 11 oder 12, wobei der Marker eine zwischen etwa 3%ige und etwa 5%ige Essigsäurelösung ist.

14. Vaginalspekulumanordnung nach einem der Ansprüche 2 bis 13, die ferner eine Lichtquelle (24) umfasst.

15. Vaginalspekulumanordnung nach einem der Ansprüche 5 bis 8, welche ferner eine Lichtquelle (24), die an dem Stützelement in der Nähe des Schaftverriegelungsmechanismus (21) befestigt ist umfasst.

16. Vaginalspekulumanordnung nach einem der Ansprüche 2 bis 15, welche ferner ein optisches Element (26) umfasst.

17. Vaginalspekulumanordnung nach Anspruch 16, wobei das optische Element (26) ein vergrößerndes optisches Element, ein fokussierbares optisches Element, einen optischen Filter oder ein Paar von Polarisatoren umfasst, einen zum Polarisieren des von der Lichtquelle emittierten Lichts und einen zum Polarisieren des vom Gewebe reflektierten Lichts, deren Polarisationsachsen senkrecht zueinander stehen.

18. Vaginalspekulumanordnung nach einem der Ansprüche 2 bis 17, wobei das Blattsystem/der Schaft (8)/die Injektionssonde (7) aus einem metallischen Material gebildet sind.

19. Vaginalspekulumanordnung nach Anspruch 18, wobei das Blattsystem/der Schaft (8) /die Injektionssonde (7) wiederverwendbar sind.

20. Vaginalspekulumanordnung nach einem der Ansprüche 2 bis 17, wobei das Blattsystem/der Schaft (8)/die Injektionsmittel (7) einen Abschnitt umfassen, der aus einer Polymerverbindung gebildet ist.

21. Vaginalspekulumanordnung nach Anspruch 20, wobei das Blattsystem/der Schaft (8)/die Injektionsmittel (7) wegwerfbar sind.

## Revendications

1. Agencement de spéculum vaginal comprenant :
un système de lames pour ouvrir le vagin ayant une première lame (1) et une seconde lame (2) pouvant être positionnées l'une par rapport à l'autre selon une pluralité d'angles et un axe de symétrie longitudinal (10) entre une partie distale et une partie proximale de chacune des première (1) et seconde (2) lames ; et
un support mécanique ayant un arbre (8) avec une première extrémité d'arbre couplée mécaniquement au système de lames et une seconde extrémité d'arbre pouvant être couplée de manière amovible à un mécanisme d'injection, un élément de support ou un appareil d'imagerie ;
**caractérisé en ce que**
ladite première extrémité d'arbre dudit arbre (8) est jointe à une jonction de poignée de lame (6) de la première lame, et une structure de la jonction de poignée de lame (6) de la seconde lame configurée pour se déplacer le long d'une structure formée le long d'un axe longitudinal dudit arbre (8).

2. Agencement de spéculum vaginal selon la revendication 1, comprenant en outre un mécanisme d'injection, dans lequel :
la seconde extrémité d'arbre est couplée de manière amovible à un mécanisme d'injection pour distribuer un marqueur de diagnostic sur la surface du tissu examiné ayant une sonde d'injection (7) ayant un axe longitudinal (11), un conteneur de marqueur (15,16) et des moyens pour permettre l'injection du marqueur (18), dans lequel les dimensions de la section transversale de la sonde d'injection (40) sont sensiblement inférieures aux dimensions de la section transversale (41) d'une ouverture arrière (42) du système de lames, et
dans lequel la position relative de l'axe longitudinal de la sonde d'injection (11) et de l'axe de symétrie longitudinal du système de lames (10) restent sensiblement fixes pour chacun de la pluralité d'angles entre les première et seconde lames, dans lequel la sonde d'injection (7) permet une application sensiblement homogène du marqueur de diagnostic sur une zone souhaitée dans le tissu vaginal ou du col de l'utérus examinée, quel que soit l'angle d'ouverture des lames (1, 2), et permet l'observation de la zone souhaitée à travers l'ouverture arrière du système de lames, avant et après l'injection du marqueur de diagnostic.

3. Agencement de spéculum vaginal selon la revendication 2, dans lequel la seconde extrémité d'arbre est couplée de manière amovible à un élément de support ou à un appareil d'imagerie.

4. Agencement de spéculum vaginal selon la revendication 2, dans lequel la sonde d'injection (7) est montée sur une partie de l'arbre (8).

5. Agencement de spéculum vaginal selon l'une quelconque des revendications 3 ou 4, dans lequel l'arbre (8) comprend un mécanisme de verrouillage.

6. Agencement de spéculum vaginal selon la revendication 3, dans lequel l'élément de support comprend un bras articulé (22) avec une première partie d'extrémité fixée à une base (23) et une seconde partie d'extrémité fixée à un mécanisme de verrouillage de l'arbre (21).

7. Agencement de spéculum vaginal selon la revendication 3 ou 4, dans lequel ledit mécanisme de verrouillage (21) comprend l'un d'un mécanisme de verrouillage mécanique ou d'un mécanisme de verrouillage magnétique ou électromagnétique.

8. Agencement de spéculum vaginal selon la revendication 5, dans lequel ladite sonde d'injection (7) est fixée à ladite base mécanique à proximité dudit mécanisme de verrouillage.

9. Agencement de spéculum vaginal selon l'une quelconque des revendications 2 à 8, dans lequel ladite sonde d'injection (7) comprend une buse.

10. Agencement de spéculum vaginal selon la revendication 9, dans lequel ladite buse (7) comprend une buse à aiguille (43).

11. Agencement de spéculum vaginal selon l'une quelconque des revendications 2 à 10, dans lequel le mécanisme d'injection comprend en outre des moyens de pompe hydraulique (15, 16, 18, 19, 20) pour pomper un volume prédéterminé d'un marqueur dans et à travers ladite sonde d'injection (7).

12. Arrangement de spéculum vaginal selon la revendication 11, dans lequel ledit volume prédéterminé du marqueur est compris entre environ 2,5 ml et environ 3,5 ml.

13. Agencement de spéculum vaginal selon la revendication 11 ou 12, dans lequel ledit marqueur est une solution d'acide acétique entre environ 3 % et environ 5 %.

14. Agencement de spéculum vaginal selon l'une quelconque des revendications 2 à 13, comprenant en outre une source de lumière (24).

15. Agencement de spéculum vaginal selon l'une quelconque des revendications 5 à 8, comprenant en outre une source de lumière (24) fixée à l'élément de support à proximité dudit mécanisme de verrouillage d'arbre (21).

16. Agencement de spéculum vaginal selon l'une quelconque des revendications 2 à 15, comprenant en outre un élément optique (26).

17. Agencement de spéculum vaginal selon la revendication 16, dans lequel l'élément optique (26) comprend un parmi un élément optique grossissant, un élément optique focalisable, un filtre optique ou une paire de polariseurs, un pour polariser la lumière émise par la source lumineuse et un pour polariser la lumière réfléchie par le tissu, ayant leurs axes de polarisation perpendiculaires l'un à l'autre.

18. Agencement de spéculum vaginal selon l'une quelconque des revendications 2 à 17, dans lequel ledit système de lames/ledit arbre (8)/ladite sonde d'injection (7) sont formés à partir d'un matériau métallique.

19. Agencement de spéculum vaginal selon la revendication 18, dans lequel ledit système de lames/ledit arbre (8)/ladite sonde d'injection (7) sont réutilisables.

20. Agencement de spéculum vaginal selon l'une quelconque des revendications 2 à 17, dans lequel ledit système de lames/ledit arbre (8)/lesdits moyens d'injection (7) comprennent une partie formée à partir d'un composé polymère.

21. Agencement de spéculum vaginal selon la revendication 20, dans lequel ledit système de lames/ledit arbre (8)/lesdits moyens d'injection (7) sont jetables.
